(19) Europäisches Patentamt
European Patent Office
Office européen des brevets

(11) **EP 3 062 919 B1**

(12) **EUROPEAN PATENT SPECIFICATION**

(45) Date of publication and mention
of the grant of the patent:
**17.06.2020 Bulletin 2020/25**

(51) Int Cl.:
***B01F 3/04*** *(2006.01)*     ***B01F 15/00*** *(2006.01)*

(21) Application number: **14856842.1**

(22) Date of filing: **03.10.2014**

(86) International application number:
**PCT/US2014/058942**

(87) International publication number:
**WO 2015/065647 (07.05.2015 Gazette 2015/18)**

(54) **MODULAR AERATION DEVICE AND METHOD**

MODULARE BELÜFTUNGSVORRICHTUNG UND VERFAHREN

DISPOSITIF D'AÉRATION MODULAIRE ET PROCÉDÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.10.2013 US 201361897246 P**

(43) Date of publication of application:
**07.09.2016 Bulletin 2016/36**

(73) Proprietor: **EMD Millipore Corporation
Burlington, MA 01803 (US)**

(72) Inventors:
• **WOOD, Amy**
**Billerica, Massachusetts 01821 (US)**
• **KRAUS, Dave**
**Billerica, Massachusetts 01821 (US)**
• **HANSEN, Anne**
**Billerica, Massachusetts 01821 (US)**
• **DER, Kara**
**Billerica, Massachusetts 01821 (US)**
• **MCSWEENEY, James**
**Billerica, Massachusetts 01821 (US)**

(74) Representative: **Uexküll & Stolberg
Partnerschaft von
Patent- und Rechtsanwälten mbB
Beselerstraße 4
22607 Hamburg (DE)**

(56) References cited:
EP-A2- 1 479 758     WO-A1-03/024578
WO-A1-2006/116067     US-A- 5 000 884
US-A- 5 007 620     US-A- 5 017 053
US-A- 5 858 283     US-A1- 2002 057 989
US-A1- 2009 145 514     US-A1- 2010 291 674
US-A1- 2011 013 473     US-A1- 2011 121 472
US-A1- 2012 313 267

## Description

This application claims priority of U.S. Provisional Application Serial No. 61/897,246 filed October 30, 2013.

## FIELD

Embodiments disclosed herein relate to a modular aeration device that can be used in a container or vessel such as a bioreactor, and in particular, such as a single use stirred tank bioreactor, as well as relating to a modular aeration device and mixing assembly, and a container or vessel containing the same.

## BACKGROUND

Traditionally, fluids such as biological materials have been processed in systems that utilize stainless steel containers or vessels. These containers are sterilized after use so that they can be reused. The sterilization procedures are expensive and cumbersome, as well as being ineffectual at times.

In order to provide greater flexibility in manufacturing and reduce the time needed to effect a valid regeneration of the equipment, manufacturers have begun to utilize disposable sterilized containers such as bags that are used once with a product batch and then disposed. An example of use of these disposable or single-use bags is in a system for mixing two or more ingredients, at least one of which is liquid and the other(s) being liquid or solid, and the bag has a mixing element or the like for causing the contents to mix as uniformly as possible.

An example of such a disposable container is a bioreactor or fermenter bag in which cells are either in suspension or on microcarriers and the container has a circulating member for circulating the liquid, gases, and in some cases the cells around the interior of the container. Many conventional mixing bags are shaped like cylinders, with the bottom of the bag forming a cone, to mimic the shape of the tanks that the disposable bags are replacing. Such a shape is conducive to mixing the contents of the bag.

Typically, the bag contains a mixer for mixing or circulating the contents, such as a magnetically coupled impeller contained within the bag and a magnetic motor outside the bag which remotely causes the impeller to spin.

The containers also can contain an aeration device or gas sparger through which gas bubbles are introduced into the container contents, such as biopharmaceutical fluids such as cell culture liquid, to exchange gases such as air, oxygen, carbon dioxide, etc. Controlled volumes of gas can be delivered to the sample. One critical aspect of an aeration device is the bubble size it produces. In bioreactor applications, for example, there is a balance between managing bubble size such that mass transfer from the gas-liquid phase or vice versa is sufficient for the process, and causing negative culture effects such as significant shear or foaming. Generally, the smaller the bubble, the more efficient the transfer of gas from the bubble to the liquid, due to increased surface area resulting from producing the multiple smaller bubbles at a given gas flow rate into the system. However, the smaller the bubble, the greater the potential damage to cells as compared to larger bubbles, and the greater the overall accumulation of foam on the liquid surface is likely to be.

Creating and maintaining a generally homogenous environment for the contents of the vessel such as cells in culture is also of critical importance in bioreactor operations. It is undesirable to have zones and/or gradients with regard to mixing (pH, nutrients, and dissolved gases), shear, temperature, etc. Some cell culture processes may require the highest possible mass transfer capabilities while others may require specific bubble sizes that are large enough that sensitive cells will remain unharmed.

US 2011/121472 A1 discloses an aeration device for infusing air into basins for growth fish. The aeration element may be in the form of a bubble generator such as a diffuser, adapted to transform the gas introduced thereto into a plurality of bubbles.

US 2011/013473 A1 discloses a disposable mixing vessel including an impeller and a sparger. The sparger is preferably a tube or a sleeve that is attached to a side wall of a container to keep the sparger line from interfering with the rotating shaft and the one or more impellers mounted on the rotating shaft.

US 2012/313267 A1 discloses an aeration device for bioreactors. Two concentric aeration elements have gas outlet openings of difference sizes.

WO 2006/116067 A1 discloses a gas sparger for container systems such as bioreactors. The sparger includes a gas permeable sparging sheet. A second sparger, configured with larger pores, can be used to remove waste products.

US 5 007 620 A discloses an apparatus for the biological processing of metal-containing ores. It comprises a plurality of diffusers, each of which includes a porous, flexible fabric membrane diffuser surface for receiving a supply of oxygen-containing gas to introduce the gas in the form of bubbles into a slurry within the container. The membrane is replaceable.

US 5 858 283 A discloses a large-area sparger comprising a flat gas-permeable diffuser bonded to an underlying layer to leave an unbounded gap that receives inflowing gas and allows inflowing gas to escape only through the diffuser.

EP 1 479 758 A2 discloses a first aeration element having a first gas permeable material having a first size different from the pore size of a second gas permeable material of a second aeration element.

WO 03/024578 A1 discloses a diffuser hose for oxygenation of water in aquaculture installations for marine organisms. Oxygen probes or sensors are used to measure the oxygen saturation.

It would be desirable to provide a container or vessel, such as a disposable or single-use container or vessel, for fluids with a versatile aeration device to aid in optimal cell culture growth performance in bioreactors, for example.

## SUMMARY

The present invention relates to an aeration device for aerating a fluid, to a system for aerating a fluid, to a method of controlling the gas delivery into a bioreactor, and to a method of regulating the mass transfer from a gas-liquid phase in a bioreactor, as defined in claims 1, 7, 8, and 10, respectively. Claim 5 relates to a bioreactor. Advantageous versions of the invention follow from the dependent claims.

Embodiments disclosed herein relate to gas spargers or aeration devices, and containers or vessels incorporating them. In accordance with certain embodiments, the aeration device comprises a plurality of aeration elements that can produce gas bubble of different sizes and deliver them to the contents (e.g., biopharmaceutical fluids) of the container. The aeration elements are interchangeable. In certain embodiments, each aeration element has a pre-selected gas permeable material that produces gas bubbles of a known size. In accordance with certain embodiments, disclosed herein are containers, such as a disposable or single-use containers, optionally having one or more inlets and one or more outlets and an optional fluid agitator or mixer associated with the container to cause mixing, dispersing, homogenizing and/or circulation of one or more ingredients contained or added to the container. In certain embodiments, the agitator assists in distributing in the fluid the gas bubbles produced by the aeration element. In accordance with certain embodiments, the container includes the aforementioned aeration device alone or in combination with the mixer.

In accordance with certain embodiments, the aeration devices disclosed herein are used in bioreactors to control the dissolved gas concentration (e.g., air, oxygen, $CO_2$, etc.) of the bioreactor contents, thereby facilitating proper growth of cell cultures in the bioreactor, or can be used in fermenters to control oxygen content of the fluid therein.

Also disclosed is a system for aerating a fluid in a container or vessel having an internal volume, the system comprising a container, an impeller assembly, a drive for the impeller assembly, and an aeration device having multiple interchangeable and removable aeration elements, the aeration device being positioned within the container internal volume to produce gas bubbles of different sizes.

Also disclosed is a method of aerating a fluid in a container or vessel with an impeller assembly and an aeration device arranged in the container. In certain embodiments, the method includes preselecting a plurality of aeration elements each having a predetermined gas permeable material of a known pore size, pore size distribution and/or total porosity and attaching each selected aeration element to a base member to assemble an aeration device. In accordance with certain embodiments, the method includes introducing a fluid into a container, wherein an impeller assembly is at least partially contained in the container, driving the blades or vanes of the impeller assembly to agitate the fluid in the container, and introducing gas into the aeration device which then produces bubbles of different, predetermined sizes to aerate the fluid in the container. In certain embodiments, the driver for the impeller assembly is external to the bag, and drives the impeller assembly magnetically.

Also disclosed is a method of controlling or regulating the mass transfer of gas into the liquid phase in a container. The method includes providing a plurality of aeration elements in the container, such as a bioreactor, each of the plurality of aeration elements being in fluid communication with a source of gas and each of the plurality of aeration elements together defining a maximum mass transfer value; and reducing that maximum mass transfer value by independently adjusting the flow of gas to each of the plurality of aeration elements. In certain embodiments, the maximum mass transfer value is reduced by stopping all flow of gas to at least one of said plurality of aeration elements. In certain embodiments, the flow of gas to each of the plurality of aeration elements is independently controllable, either manually or via a controller such as a PLC. In certain embodiments, a single gas source is used, and is independently manifolded to each individual aeration element such as with suitable tubing or the like, either externally of the container or internally in the container.

The modular approach to the aeration device provides manufacturing benefits, since overmolding multiple smaller sections of gas permeable material in each modular element reduces complexity and lowers mold costs compared to a process that requires overmolding of one large gas permeable section.

## BRIEF DESCRIPTION OF THE DRAWINGS

FIG. 1 is an exploded view of an aeration device and mixing assembly in accordance with certain embodiments;

FIG. 2A is a top view of an impeller cup in accordance with certain embodiments;

FIG. 2B is a cross-sectional view taken along line 2-2 of FIG. 2A;

FIG. 3A is a top view of an aeration element in accordance with certain embodiments;

FIG. 3B is a cross-sectional view of an aeration element taken along line A-A of FIG. 3A;

FIG. 3C is a cross-sectional view of an aeration element taken along line F-F of FIG. 3A;

FIG. 3D is a top view of a tab of an aeration element in accordance with certain embodiments;

FIG. 3E is a cross-sectional view of a tab taken along line E-E of FIG. 3D;

FIG. 3F is an enlarged view of detail B in FIG. 3B showing a gas channel in accordance with certain embodiments;

FIG. 4A is a perspective view of an aeration device and mixing assembly in accordance with certain embodiments;

FIG. 4B is a cross-sectional view of the aeration device and mixing assembly of FIG. 4A;

FIG 4C is a cross-sectional view of the mixing assembly in accordance with certain embodiments;

FIG. 5 is a bottom view of an aeration element in accordance with certain embodiments;

FIG. 6 is a cross-sectional view of an aeration element taken along line D-D of FIG. 5;

FIG. 7 is a graph of the characterization of $k_L a$ for spargers sections from 1 to 4 and full, with increasing air flow rate;

FIG. 8 is a graph of gas transfer effectiveness v. area;

FIG. 9 is a graph of average gas transfer effectiveness v. area;

FIG. 10 is a graph of air flow necessary to achieve 30 $hr^{-1}$ $k_L a$; and

FIG. 11 is a graph of air flow rate v. $k_L a$.

## DETAILED DESCRIPTION

In accordance with certain embodiments, the disposable or single-use container designed to receive and hold a fluid is not particularly limited, and can be formed of monolayer or multilayer flexible walls formed of a polymeric composition such as polyethylene, including ultrahigh molecular weight polyethylene, linear low density polyethylene, low density or medium density polyethylene; polyproplylene; ethylene vinyl acetate (EVOH); polyvinyl chloride (PVC); polyvinyl acetate (PVA); ethylene vinyl acetate copolymers (EVA copolymers); blends of various thermoplastics; co-extrusions of different thermoplastics; multilayered laminates of different thermoplastics; or the like. By "different" it is meant to include different polymer types such as polyethylene layers with one or more layers of EVOH as well as the same polymer type but of different characteristics such as molecular weight, linear or branched polymer, fillers and the like. Typically medical grade and preferably animal-free plastics are used. They generally are sterilizable such as by steam, ethylene oxide or radiation such as beta or gamma radiation. Most have good tensile strength, low gas transfer and are either transparent or at least translucent. Preferably the material is weldable and is unsupported. Preferably the material is clear or translucent, allowing visual monitoring of the contents. The container can be provided with one or more inlets, one or more outlets and one or more optional vent passages.

In certain embodiments, the container may be a disposable, deformable, foldable, flexible bag that defines a closed internal volume, that is sterilizable for single-use, capable of accommodating contents, such as biopharmaceutical fluids, in a fluid state, and that can accommodate a mixing device partially or completely within the interior volume, and an aeration device within the interior volume. In certain embodiments, the closed volume can be opened, such as by suitable valving, to introduce a fluid into the volume, and to expel fluid therefrom, such as after mixing or other processing is complete.

In certain embodiments, the container may be a two-dimensional or "pillow" bag, or it may be a three-dimensional bag. The particular geometry of the container is not particularly limited. In certain embodiments, the container may include a rigid base, which provides access points such as ports or vents. Each container may contain one or more inlets and outlets and optionally other features such as sterile gas vents and ports for the sensing of the liquid within the container for parameters such as conductivity, pH, temperature, dissolved gases and the like.

In certain embodiments, each container can contain, either partially or completely within its interior, an impeller assembly for mixing, dispersing, homogenizing, and/or circulating one or more liquids, gases and/or solids contained in the container. In accordance with certain embodiments, the impeller assembly may include one or more blades or vanes, which are movable, such as by rotation or oscillation about an axis. In certain embodiments, the impeller assembly converts rotational motion into a force that mixes the fluids it is in contact with. In certain embodiments, the blades are made of plastic.

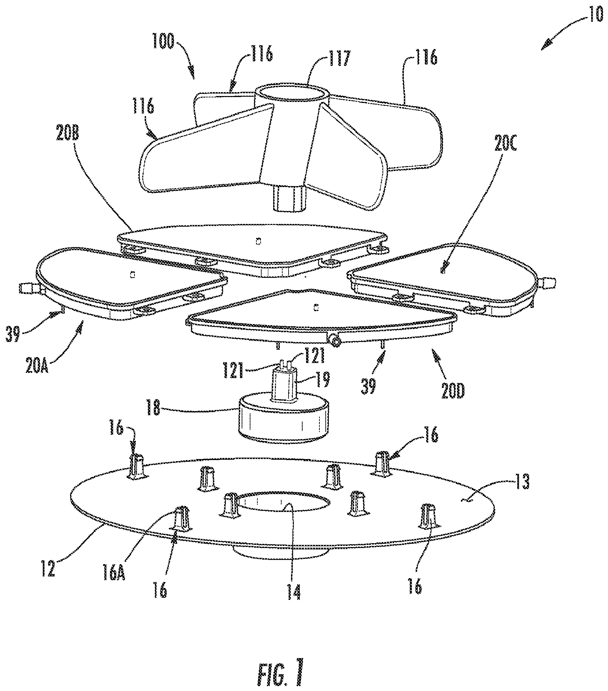

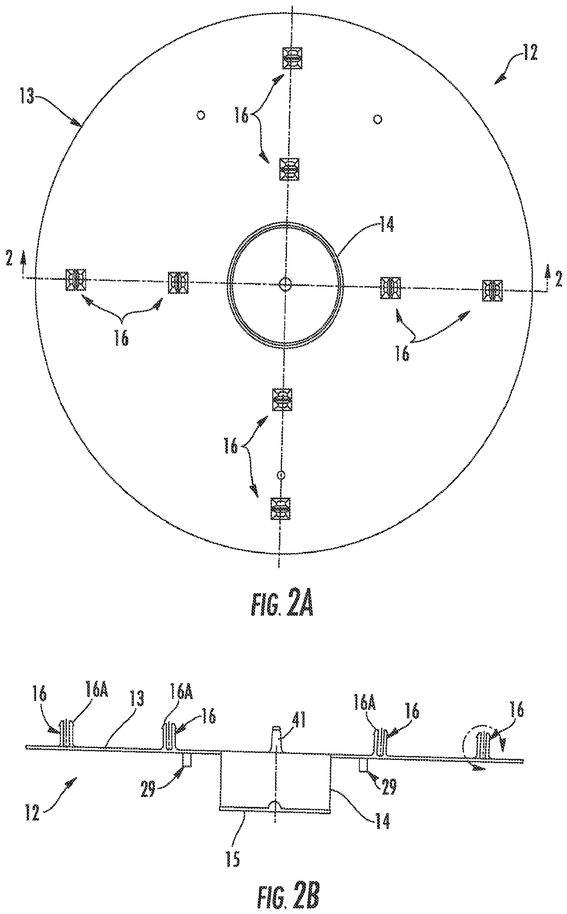

Turning now to FIG. 1, there is shown an aeration device 10 in accordance with certain embodiments. The device 10 includes an impeller cup 12 that in the illustrative embodiment shown, is a disc or circularly-shaped rigid base member 13 having a central cylindrical cup 14 that terminates in a bottom 15, best seen in FIGS. 2A and 2B. The cup 14 is configured to receive an overmolded magnet 18 used in driving the impeller. A plurality of spaced projections, rods, cones or pins 16 extend upwardly from the top surface of the base member 13. In the embodiment shown, there are 8 such projections, linearly aligned in pairs, although the number and location of the projections on the base member are not particularly limited. The projections are configured and arranged to engage with corresponding tabs in the aeration elements as discussed in greater detail below. In certain embodiments, as best seen in FIG. 2B, each projection includes spaced body members each terminating in a head portion 16A that flares outwardly as shown. In certain embodiments, one or more legs 29 extend downwardly from the bottom surface of the base member 13 (FIG. 2B) and can be received by corresponding respective receiving holes (not shown) in the housing or tank to position the device appropriately so that an external impeller drive may be connected. The base member 13 and the aeration elements can be made of plastic. The base member 13 acts as a support member or substrate for the modular aeration elements, and removably and selectively attaches to each of the aeration elements. In certain embodiments, the base member 13 is common to all of the aeration elements.

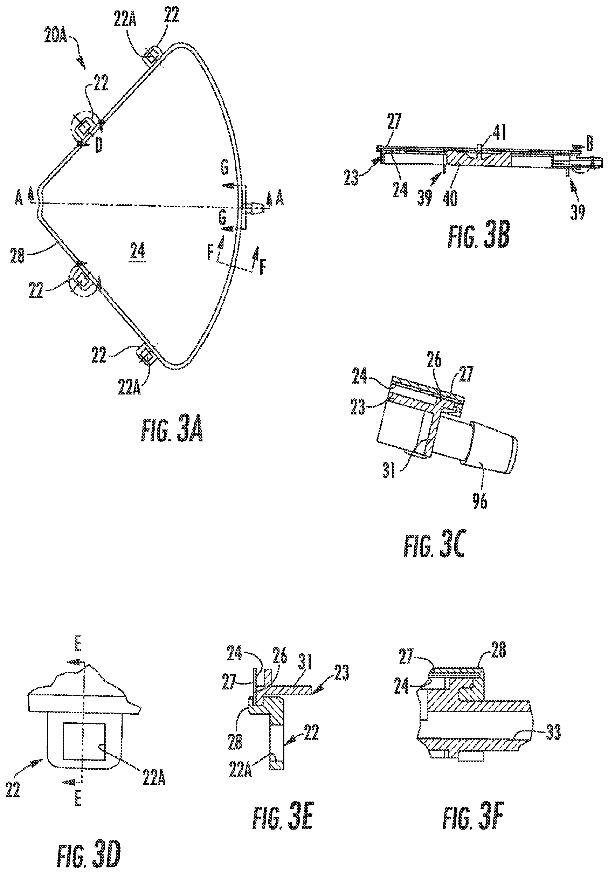

FIG. 1 also shows a plurality of aeration elements 20A-20D. In the embodiment shown, four such aeration elements are depicted, although fewer or more could be used. As best seen in FIGS. 3A-3F and 5-6, in the embodiment shown each aeration element is generally pie shaped, and includes a perimeter flange 28 that is C-shaped in cross-section (FIG. 3E). The flange 28 carries one or more perimeter tabs 22, each extending outwardly from the perimeter and having an aperture 22A configured and positioned to releasably engage with a respective projection 16 in the base member 13, such as by a snap fit. In certain embodiments, the diameter of each aperture 22A increases from the top opening towards the bottom opening, i.e., it flares radially outwardly as can be seen in FIG. 3E. Each aeration element can be readily engaged and disengaged with the impeller cup 12, by aligning each aperture 22A in each tab 22 with a corresponding pin 16 in the base member 13, enabling selection of the desired bubble size vs. mass transfer capabilities simply by selecting and attaching an aeration element with the desired specifications.

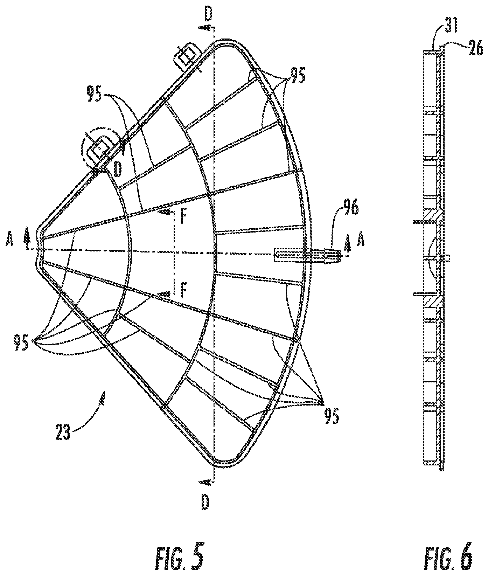

In certain embodiments, each aeration element includes a lower plate member 23, which has a perimeter side wall 31 having a flange 26 extending radially outwardly. As seen in FIGS. 5 and 6, the lower plate member 23 of the aeration element may include a plurality of stiffening ribs 95 ribs arranged in a grid-like pattern to provide added strength. The lower plate member 23 mate with the top plate member to define there between a closed cavity (but for the gas permeable material 24) into which gas is introduced via connecting member 96. The aeration element may include a screen 27 such as woven monofilament fabric material available from Sefar Filtration Inc., such as PETEX 07-350/34 having mesh openings of 350 $\mu$m. The aeration element may also include a sheet or film 24 of a gas permeable material. Suitable materials include polymeric films and sheets, including but not limited to spunbond olefin materials such as Tyvek® 1059B, polytetrafluoroethylene (TEFLON®), polysulfone, polypropylene, silicone, fluoropolymers such as polyvinylidene fluoride (KYNAR®), POREX® membranes such as POREX® 4903, RM membranes commercially available from EMD Millipore, etc.

In certain embodiments, the gas permeable material is overmolded into place, such as onto perimeter flange 26 of the side wall 31 of plate 23, and can be sandwiched by the top perimeter flange member 28 (FIG. 3E). The screen 27 can be placed on top of the gas permeable material 24 and also sandwiched by the top flange member 28.

Each aeration element may include one or more legs 39 extending downwardly to selectively elevate each aeration element above the impeller cup. This eliminates variable gap heights.

Each aeration element includes a dedicated inlet gas source, including a channel 33 (FIG. 3F) that can be placed in fluid communication with a gas source (not shown) such as with a hose, tube, conduit or the like, via connecting member 96, for example. The channel provides fluid communication from the gas source to the gas permeable material via the channel 33.

As seen in FIG. 3B, in certain embodiments there is a concave section 40 in the plate member 23 for the injection location in the mold, and an axial protrusion 41 that is a gate vestige that will be removed before overmolding.

The aeration device thus includes a plurality of separate aeration elements, for example quarter circles as illustrated, each containing its own inlet gas source, and each capable of receiving a customized or pre-selected gas permeable material of a predetermined pore size, allowing for customization of pore size, bubble size, and total surface area of gas permeable material within a single-use container such as a bag.

The aeration device efficiency for distribution of uniform bubbles is improved from the conventional single aeration device with a single gas inlet with a surface area of X, by including multiple gas inlets into multiple aeration elements which together add to a total surface area X. This approach of breaking down the aeration device to modular sections allows the device of a certain material and specified total surface area to use that total surface area more efficiently. Gas dispersion within each aeration element fed by a dedicated gas inlet enables more even distribution across the total surface area of all aeration elements. This is particularly the case when the surface that the sparger is on is not horizontal. This helps to keep the bubble size produced by the gas permeable material more consistent, the location of the generation of bubbles in relation to the mixing element more consistent, in some cases narrows the size distribution of the bubbles produced by the sparging element, and results in a more homogenous environment for the processed fluid such as cell cultures.

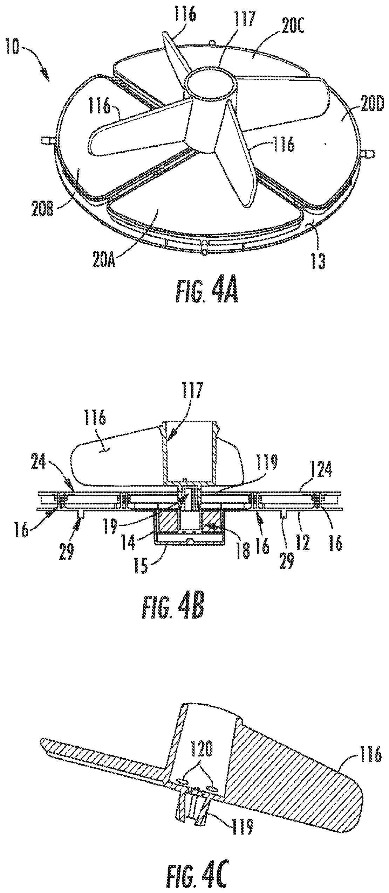

In certain embodiments, as the bubbles emerge from each aeration element 20A-20D, they are dispersed in the vessel by a mixing assembly 100. In certain embodiments, the mixing assembly 100 is centrally located with respect to the aeration elements, and is positioned above the aeration elements with respect to the direction of gas bubble emission from the aeration elements (FIG. 4A).

In certain embodiments, the mixing assembly 100 is an impeller assembly having one or more moveable blades or vanes 116, with four spaced blades 116 shown in FIGS. 1 and 4A for purposes of illustration. The number and shape of the blades 116 is not particularly limited, provided they provide sufficient agitation of the fluid within the container when actuated. The blade or blades may be constructed of plastic material, such as polyethylene, or any polymer resistant to gamma irradiation, such as a polypropylene co-polymer. In certain embodiments, the blades 116 are each attached to a central cylindrical member 117, seen in cross-section in FIG. 4B and 4C, which has an axially extending lower cylindrical member 119, open at its bottom end, which receives the connector 19 of overmolded magnet 18. One or more apertures 120 are provided to receive pins 121 of the overmolded magnet 18, which are then heated and deformed to permanently couple the magnet to the impeller.

The blades 116 are positioned axially above the overmolded magnet 18 as well as above the aeration elements 20A-20D, where they are free to rotate when the magnetic impeller is drive by a suitable actuator. Maintaining the consistent location of the aeration device under the impeller assembly enables better distribution of the gas into the volume of the container as each modular aeration element is positioned equally or symmetrically under the impeller. This can maintain a smaller distribution of bubble sizes produced, since the interaction in the high-shear impeller zone can impact bubble size, formation and behavior.

In certain embodiments, when the impeller assembly 100 is installed in a container, the cylindrical cup 14 that houses the overmolded magnet 18 protrudes outside the container and is sealed to the container. In this embodiment, the remainder of the impeller assembly 100 is housed inside the internal volume of the container. Preferably the aeration device and mixing assembly is positioned at or near the bottom of the container, when the container is in mixing position (such as a hanging position) and in close proximity to an inlet of the container. Thus, in certain embodiments, at least a portion of the impeller assembly is internal to the container, and the driver for the impeller assembly is external to the container.

The modular feature of the aeration elements 20A-20D allows flexibility in delivering a different range of bubble size versus mass transfer capability. For example, if only three aeration elements (e.g., 20A, 20B and 20C) are used instead of four (e.g., 20D is not used), the mass transfer capability of the device can be modified without changing the bubble size and without building a new device, since each aeration device has a dedicated gas feed. The control of which of the plurality of aeration devices receives gas feed from one or more sources of gas can be carried out manually or with a controller such as a PLC. Customization of aeration elements at the time of final assembly without impact on manufacturing of the container is achieved, as well as improvement in the ability to control and manage the shear produced by bubbles within a container such as a bioreactor, due to the improved management of bubble size and bubble velocity upon exiting each aeration element. Deleterious foam production also may be reduced. Tubing for supplying gas to the aeration device can be manifolded internal to the container or external to the container, regardless of whether a single gas source or multiple gas sources are used, allowing for ease of use for the particular application with flexibility of design. Each aeration element can be manifolded individually, thereby providing greater control over gas delivery into the system.

In certain embodiments, feedback control loops are employed in order to maintain a desired dissolved gas concentration, for example oxygen or carbon dioxide, within the bioreactor/fermentor broth or system. The controlling system typically receives a signal input representing the real time process value from a probe/sensor which is in or on line, triggering a response output, as determined by a control loop algorithm which is built to provide action such as altering the gas composition and/or flow rate into the aeration device to achieve the desired effect on the dissolved gas process value. Dissolved gas (e.g., $O_2$) can monitored on a continuous or continual basis, and the flowrate adjusted via the feedback control loop on a continuous or continual basis.

Depending upon how the aeration device is manifolded, the control system can be managed to include response outputs involving various manifolding techniques that could vary the number of aeration devices within the plurality of aeration devices as part of the feedback control algorithm designed to manage a specific, desired, dissolved gas concentration within the bioreactor/fermentor system.

Increasing aeration element surface area by employing a plurality of spargers with individual air source inlets can increase the volumetric mass transfer coefficient $k_L a$ capability of the system. The $k_L a$ can be assessed via the static gassing out method, where the system is purged of oxygen through the addition of nitrogen gas. Air is then added at a controlled rate (with agitation at a controlled speed). A record of dissolved oxygen concentration over time is plotted and a mathematical analysis is performed to fine $k_L a$ according to the following formula:

$$ ln \left[ \frac{(C^* - C_{t1})}{(C^* - C_{t2})} \right] = k_L a * t $$

where C = DO concentration, t = time

Under certain conditions, employing four aeration elements with individual gas inlet sources provides additional kLa than an element designed with a single (equal total) surface area supplied with gas from a single inlet.

EXAMPLE 1

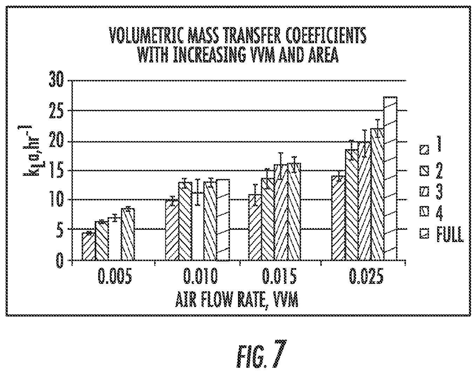

A series of $k_L a$ trials was run in a 1000L vessel, fitted with a 330.2 mm (13") rounded impeller, a baffle and appropriate sensors, to establish the relationship between area of gas permeable material in the modular sparger and expected gas transfer efficiency. For these trials, impeller rpm was kept constant at 60 rpm, a power input of 10 W/m$^3$. Three replicates were run at each condition, using Tyvek® 1059B as the gas permeable material in all four positions of the modular sparger of FIG. 1, and air flow rates from 5 lpm to 20 lpm, for air flow rates (vvm) from 0.005 to 0.020 min$^{-1}$. A full size sparger was also tested. Mock media was used, consisting of water, Pluronic (0.2%), 1X PBS, and 50 ppm antifoam. The results are shown in FIG. 7. The data indicate that higher air flow rates result in higher $k_L a$ values.

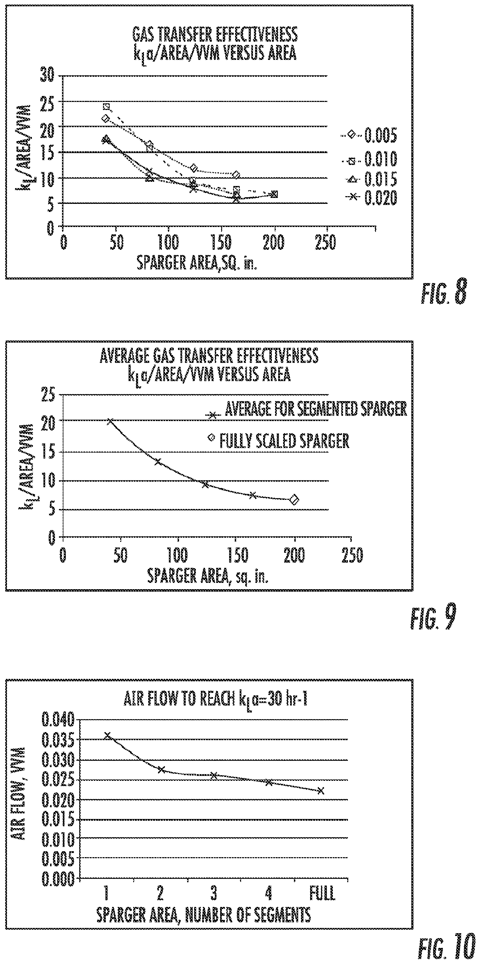

Gas transfer effectiveness can be ascertained by comparing $k_L a$/area/vvm to the area of each modular segment of the sparger. FIG. 8 shows this relationship based on an area of each module of the modular sparger of 40.98 square inches, and an area of a full size sparger of 200 square inches. FIG. 9 shows the average gas transfer effectiveness across all air flow rates.

Since the $k_L a$/area/vvm value increases with smaller sparger area, it is clear that the modular sparger provides better efficiency in gas transfer; a smaller area can be used to achieve the same $k_L a$ without an increase in air flow. This is shown in FIG. 10. The plot in FIG. 10 shows that the necessary air flow for a $k_L a$ of 30 hr-1 is about 0.025 vvm (25 lpm for 1000L). Using just one module, the air flow requirement to achieve this same $k_L a$ rises to 0.035 vvm. Accordingly, two modules of Tyvek® can be used at about 0.025 vvm air flow to achieve the desired $k_L a$ value of 30 hr$^{-1}$.

EXAMPLE 2

The modular sparger in accordance with embodiments disclosed herein allows for more than one type of gas permeable material to be used in the system. EXAMPLE 1 demonstrates that high $k_L a$ values can be achieved where TYVEK® material occupies only two of the four modular segments of the sparger. For example, the gas permeable material for the remaining two modular segments could be chosen to produce bubbles larger than those produced using TYVEK® material, such as Porex® POR97619 ("PE-10"), POR4920 (PE-40") and POR 4903 ("PE-90"), all made of polyethylene.

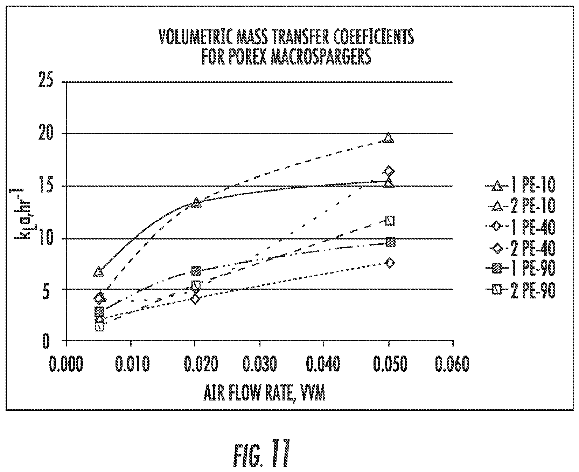

Using the vessel of EXAMPLE 1, these three types of gas permeable material were evaluated in one and two segments of the sparger, over a range of air flow rates from 5 lpm to 50 lpm (0.005 vvm to 0.05 vvm), with impeller rpm kept constant at 60 rpm. A summary of the results is shown in FIG. 11.

In general, the larger pore size (PE-90) material tended to give lower $k_L a$ values, while the smallest pores (PE-10) gave the highest $k_L a$ values. Lower kLa values can, however, be tolerated where larger bubble size is desired.

**Claims**

1. An aeration device for aerating a fluid within a bioreactor, comprising:

   a base member (13);
   a plurality of separate, interchangeable aeration elements (20A-20D) each removably attached to said base member (13), each said aeration element (20A-20D) comprising a gas permeable material, which comprises a membrane, and each said aeration element (20A-20D) having an inlet adapted to connect to a gas source, each said inlet being in fluid communication with said gas permeable material;
   wherein said plurality of separate, interchangeable aeration elements (20A-20D) comprises first and second aeration elements, said first aeration element having a first gas permeable material having a first pore size, and said second aeration element having a second gas permeable material having a second pore size different from said first pore size.

2. The aeration device of claim 1, wherein said gas permeable material comprises a spunbond olefin material.

3. An aeration and mixing assembly, comprising an aeration device (10) according to any of the preceding claims and a mixing device,

   said mixing device comprising:
   an impeller assembly (100) comprising at least one movable blade (116).

4. The aeration and mixing assembly of claim 3, wherein said impeller assembly (100) is magnetically driven.

5. A bioreactor for processing a fluid sample, comprising:

   an internal volume;
   an aeration and mixing assembly (10, 100) according to claim 3,
   wherein said aeration device (10) is positioned in said internal volume, and
   wherein said mixing device (100) is at least partially positioned in said internal volume.

6. The bioreactor of claim 5, wherein said bioreactor is formed of a flexible material.

7. A system for aerating a fluid within a bioreactor comprising:

   a bioreactor having an internal volume;

an impeller assembly (100) at least partially within said internal volume;

a drive for said impeller assembly (100); and

a fluid aeration device (10) in said internal volume and having multiple separate interchangeable fluid aeration elements (20A-20D) mounted on a base member (13), each fluid aeration element (20A-20D) having pores of different sizes than the other aeration elements, the fluid aeration device (10) being positioned within the bioreactor internal volume to produce gas bubbles of different sizes when said device is in fluid communication with a source of gas.

8. A method of controlling the gas delivery into a bioreactor, comprising:

providing the fluid aeration device (10) of claim 1 in said bioreactor; and

supplying gas from said gas source to fewer than all of said plurality of said aeration elements (20A-20D).

9. The method of claim 8, wherein there are four aeration elements (20A-20D), and wherein said supplying step supplies gas from said gas source to fewer than said four aeration elements.

10. A method of regulating the mass transfer from a gas-liquid phase in a bioreactor containing a biopharmaceutical fluid, comprising:

providing the fluid aeration device (10) of claim 1 in said bioreactor, each of said plurality of aeration elements (20A-20D) being in independent fluid communication with a source of gas, said plurality of aeration elements (20A-20D) together defining a maximum mass transfer value;

sensing the dissolved gas concentration in said bioreactor; and

reducing said maximum mass transfer value in response to said sensed concentration

- by independently adjusting the flow of gas to one or more of said plurality of aeration elements (20A-20D),

- or by supplying gas to fewer than all of said plurality of aeration elements (20A-20D).

**Patentansprüche**

1. Belüftungsvorrichtung zum Belüften eines Fluids in einem Bioreaktor, umfassend:

ein Basiselement (13);

eine Vielzahl von getrennten, austauschbaren Belüftungselementen (20A-20D), die jeweils entfernbar an dem Basiselement (13) angebracht sind, wobei jedes Belüftungselement (20A-20D) ein gasdurchlässiges Material umfasst, das eine Membran umfasst, und jedes Belüftungselement (20A-20D) einen Einlass aufweist, der zur Verbindung mit einer Gasquelle angepasst ist, wobei jeder Einlass in Fluidverbindung mit dem gasdurchlässigen Material steht;

wobei die mehreren getrennten austauschbaren Belüftungselemente (20A-20D) erste und zweite Belüftungselemente umfassen, wobei das erste Belüftungselement ein erstes gasdurchlässiges Material mit einer ersten Porengröße und das zweite Belüftungselement ein zweites gasdurchlässiges Material mit einer zweiten Porengröße aufweist, die sich von der ersten Porengröße unterscheidet.

2. Belüftungsvorrichtung nach Anspruch 1, wobei das gasdurchlässige Material ein im Spinnvliesverfahren hergestelltes Olefinmaterial umfasst.

3. Belüftungs- und Mischanordnung, umfassend eine Belüftungsvorrichtung (10) nach einem der vorhergehenden Ansprüche und eine Mischvorrichtung,

wobei die Mischvorrichtung umfasst:
eine Laufradanordnung (100), die mindestens eine bewegliche Schaufel (116) umfasst.

4. Belüftungs- und Mischanordnung nach Anspruch 3, wobei die Laufradanordnung (100) magnetisch angetrieben ist.

5. Bioreaktor zur Verarbeitung einer Fluidprobe, umfassend:

ein Innenvolumen;

eine Belüftungs- und Mischanordnung (10, 100) gemäß Anspruch 3,

wobei die Belüftungsvorrichtung (10) in dem Innenvolumen angeordnet ist und

wobei die Mischvorrichtung (100) zumindest teilweise in dem Innenvolumen angeordnet ist.

6. Bioreaktor nach Anspruch 5, wobei der Bioreaktor aus einem flexiblen Material gebildet ist.

7. System zum Belüften eines Fluids in einem Bioreaktor, umfassend:

einen Bioreaktor mit einem Innenvolumen;

eine Laufradanordnung (100) zumindest teilweise innerhalb des Innenvolumens;

einen Antrieb für die Laufradanordnung (100); und

eine Fluidbelüftungsvorrichtung (10) in dem Innenvolumen und mit mehreren getrennten austauschbaren Fluidbelüftungselementen (20A-20D), die auf einem Basiselement (13) angebracht sind, wobei jedes Fluidbelüftungselement (20A-20D) Poren mit verschiedener Größe als die anderen Belüftungselemente aufweist, die Fluidbelüftungsvorrichtung (10) innerhalb des Innenvolumens des Bioreaktors angeordnet ist, um Gasblasen unterschiedlicher Größe zu erzeugen, wenn die Vorrichtung in Fluidverbindung mit einer Gasquelle steht.

8. Verfahren zum Steuern der Gaszufuhr in einen Bioreaktor, umfassend:

   Bereitstellen der Fluidbelüftungsvorrichtung (10) gemäß Anspruch 1 in dem Bioreaktor; und Zuführen von Gas von der Gasquelle zu weniger als der gesamten Vielzahl der Belüftungselemente (20A-20D).

9. Verfahren nach Anspruch 8, wobei vier Belüftungselemente (20A-20D) vorhanden sind und wobei der Zufuhrschritt Gas von der Gasquelle zu weniger als den vier Belüftungselementen liefert.

10. Verfahren zum Regulieren des Stoffübergangs von einer Gas-Flüssig-Phase in einem Bioreaktor, der ein biopharmazeutisches Fluid enthält, umfassend:

    Bereitstellen der Fluidbelüftungsvorrichtung (10) gemäß Anspruch 1 in dem Bioreaktor, wobei jedes der mehreren Belüftungselemente (20A-20D) in unabhängiger Fluidverbindung mit einer Gasquelle steht, wobei die mehreren Belüftungselemente (20A-20D) zusammen einen maximalen Massetransferwert definieren; Erfassen der Konzentration des gelösten Gases in dem Bioreaktor; und Reduzieren des maximalen Massetransferwerts als Reaktion auf die erfasste Konzentration

    - durch unabhängiges Einstellen des Gasstroms auf eines oder mehrere der mehreren Belüftungselemente (20A-20D)
    - oder durch Zuführen von Gas zu weniger als allen mehreren Belüftungselementen (20A-20D).

**Revendications**

1. Dispositif d'aération pour aérer un fluide dans un bioréacteur, comprenant :

   un organe de base (13) ;
   une pluralité d'éléments d'aération distincts et interchangeables (20A-20D), tous fixés de manière amovible audit organe de base (13), chacun desdits éléments d'aération (20A-20D) comprenant un matériau perméable aux gaz, qui comprend une membrane, et chacun desdits éléments d'aération (20A-20D) ayant une entrée adaptée pour se connecter à une source de gaz, chacune desdites entrées étant en communication fluide avec ledit matériau perméable aux gaz ;
   dans lequel ladite pluralité d'éléments d'aération distincts et interchangeables (20A-20D) comprend des premier et deuxième éléments d'aération, ledit premier élément d'aération comportant un premier matériau perméable aux gaz ayant une première taille de pore, et ledit deuxième élément d'aération comportant un deuxième matériau perméable aux gaz ayant une deuxième taille de pore, différente de ladite première taille de pore.

2. Dispositif d'aération selon la revendication 1, dans lequel ledit matériau perméable aux gaz comprend un matériau oléfinique filé-lié.

3. Ensemble d'aération et de mélangeage, comprenant un dispositif d'aération (10) selon l'une quelconque des revendications précédentes et un dispositif mélangeur,

   ledit dispositif mélangeur comprenant :
   un ensemble de rouet (100) comprenant au moins une aube mobile (116).

4. Ensemble d'aération et de mélangeage selon la revendication 3, dans lequel ledit ensemble de rouet (100) est entraîné de façon magnétique.

5. Bioréacteur permettant de traiter un échantillon fluide, comprenant :

   un volume intérieur ;
   un ensemble d'aération et de mélangeage (10, 100) selon la revendication 3,
   dans lequel ledit dispositif d'aération (10) est positionné dans ledit volume intérieur, et
   dans lequel ledit dispositif mélangeur (100) est au moins en partie positionné dans ledit volume intérieur.

6. Bioréacteur selon la revendication 5, dans lequel ledit bioréacteur est fait d'un matériau flexible.

7. Système permettant d'aérer un fluide dans un bioréacteur comprenant :

   un bioréacteur ayant un volume intérieur ;
   un ensemble de rouet (100) au moins partiellement dans ledit volume intérieur ;

un entraînement pour ledit ensemble de rouet (100) ; et

un dispositif d'aération de fluide (10) dans ledit volume intérieur et comportant de multiples éléments d'aération de fluide distincts et interchangeables (20A-20D) montés sur un organe de base (13), chaque élément d'aération de fluide (20A-20D) ayant des pores d'une taille différente des autres éléments d'aération, le dispositif d'aération de fluide (10) étant positionné dans le volume intérieur du bioréacteur pour produire des bulles de gaz de différentes tailles quand ledit dispositif est en communication fluide avec une source de gaz.

8. Procédé de commande de la distribution de gaz dans un bioréacteur, comprenant les étapes suivantes :

placer le dispositif d'aération de fluide (10) de la revendication 1 dans ledit bioréacteur ; et

fournir du gaz provenant de ladite source de gaz à un nombre inférieur à l'intégralité desdits éléments d'aération (20A-20D).

9. Procédé selon la revendication 8, dans lequel il y a quatre éléments d'aération (20A-20D), et dans lequel ladite étape de fourniture fournit du gaz provenant de ladite source de gaz à moins de quatre desdits éléments d'aération.

10. Procédé de régulation du transfert de masse à partir d'une phase gazeuse-liquide dans un bioréacteur contenant un fluide biopharmaceutique, comprenant les étapes suivantes :

placer le dispositif d'aération de fluide (10) de la revendication 1 dans ledit bioréacteur, chacun desdits éléments d'aération (20A-20D) étant en communication fluide indépendante avec une source de gaz, lesdits éléments d'aération (20A-20D) définissant ensemble une valeur de transfert de masse maximale ;

mesurer la concentration en gaz dissous dans ledit bioréacteur ; et

réduire ladite valeur de transfert de masse maximale en réponse à ladite concentration mesurée

- en ajustant indépendamment le débit de gaz vers l'un ou plusieurs desdits éléments d'aération (20A-20D),

- ou en fournissant du gaz à un nombre inférieur à l'intégralité desdits éléments d'aération (20A-20D).

FIG. 1

FIG. 2A

FIG. 2B

FIG. 3A

FIG. 3B

FIG. 3C

FIG. 3D

FIG. 3E

FIG. 3F

FIG. 4A

FIG. 4B

FIG. 4C

FIG. 5

FIG. 6

FIG. 7

*FIG. 8*

*FIG. 9*

*FIG. 10*

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61897246
- US 2011121472 A1
- US 2011013473 A1
- US 2012313267 A1
- WO 2006116067 A1
- US 5007620 A
- US 5858283 A
- EP 1479758 A2
- WO 03024578 A1